# EUROPÄISCHE PATENTANMELDUNG

(11) **EP 4 053 576 A1**
(43) Veröffentlichungstag der Anmeldung: **07.09.2022**
(21) Anmeldenummer: 21161008.4
(22) Anmeldetag: 05.03.2021
(51) Int. Cl.: G01R 33/28, G01R 33/54

(54) **VERFAHREN ZUR ERMITTLUNG EINES SIMULATIONSWERTES FÜR EINE MR-MESSUNG, EINE RECHENEINHEIT, EIN SYSTEM UND EIN COMPUTERPROGRAMMPRODUKT**

(71) Anmelder: Siemens Healthcare GmbH, 91052 Erlangen (DE)
(72) Erfinder: Gebhardt, Matthias, 91052 Erlangen (DE); Zeller, Mario, 91054 Erlangen (DE)

(57) **Zusammenfassung**

Die Erfindung betrifft ein Verfahren zur Ermittlung eines eine sicherheitsrelevante Größe beschreibenden Simulationswertes für eine MR-Messung, eine Recheneinheit, ein System und ein Computerprogrammprodukt.

Gemäß dem Verfahren wird eine MR-Pulssequenz bereitgestellt, die ausgebildet ist, anhand ihrer eine MR-Messung eines Patienten mit einem MR-Scanner durchzuführen, wobei die MR-Pulssequenz eine zeitliche Abfolge von mehreren HF-Pulsen und mehreren Gradientenpulsen umfasst. Zudem wird zumindest ein Patientenwert bereitgestellt, der eine Eigenschaft des Patienten beschreibt. Anhand der MR-Pulssequenz und des zumindest einen Patientenwertes wird durch eine Recheneinheit zumindest ein Simulationswert ermittelt. Der zumindest eine Simulationswert beschreibt eine sicherheitsrelevante Größe bei einer Durchführung einer MR-Messung anhand der MR-Pulssequenz. Bei der Ermittlung des zumindest einen Simulationswertes werden spezifische Eigenschaften der HF-Pulse und der Gradientenpulse der MR-Pulssequenz sowie deren zeitliche Abfolge berücksichtigt. Ferner wird der zumindest eine Simulationswert bereitgestellt.

## Beschreibung

Die Erfindung betrifft ein Verfahren zur Ermittlung eines eine sicherheitsrelevante Größe beschreibenden Simulationswertes für eine MR-Messung, eine Recheneinheit, ein System und ein Computerprogrammprodukt.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Hierbei wird typischerweise ein menschlicher oder tierischer Patient in einem Untersuchungsraum eines MR-Scanners positioniert. Während einer MR-Messung werden üblicherweise mit Hilfe einer Hochfrequenzantenneneinheit des MR-Scanners hochfrequente (HF) Pulse in das Untersuchungsobjekt eingestrahlt. Durch den HF-Puls wird ein magnetisches Wechselfeld, ein sogenanntes B1-Feld, in dem Untersuchungsraum erzeugt. Davon zu unterscheiden ist ein statisches Hauptmagnetfeld, auch B0-Feld genannt. Ferner werden mit Hilfe einer Gradientenspuleneinheit des MR-Scanners Gradientenpulse geschalten, wodurch temporäre Magnetfeldgradienten im Untersuchungsraum erzeugt werden. Durch die erzeugten Pulse werden im Patienten ortskodierte MR-Signale angeregt und ausgelöst. Die MR-Signale werden von dem MR-Scanner empfangen und zur Rekonstruktion von MR-Abbildungen verwendet.

Beim Betrieb von MR-Scannern sind üblicherweise diverse normative Vorgaben bezüglich einer spezifischen Absorptionsrate (SAR) und/oder eines B1+rms-Wertes bei einem Einstrahlen der HF-Pulse und/oder einer Stimulation des Patienten durch ein Schalten der Gradientenpulse einzuhalten. Falls der Patient ein Implant aufweist, sind üblicherweise besonders strenge Vorgaben einzuhalten. Aber auch zum Schutz kritischer Komponenten des MR-Scanners, wie z.B. von HF-Verstärkern, kann es notwendig sein, die Leistung der HF-Einstrahlung und/oder die zeitliche Veränderung der durch die Gradientenspueneinheit fließenden Ströme zu begrenzen, um beispielsweise eine zu starke Komponentenerwärmung zu verhindern.

Gängige Sicherheitsarchitekturen umfassen hierbei eine Echtzeit-Überwachung während der MR-Messung ermittelter Messgrößen, die mit der Sendeaktivität der Hochfrequenzantenneneinheit und/oder der Aktivität der Gradientenspuleneinheit korreliert. Im Falle einer Überschreitung wird die MR-Messung dann automatisch abgebrochen. Solche Abbrüche sollten allerdings im klinischen Betrieb der Ausnahmefall bleiben, führen sie doch nicht nur zu Frustration der Patienten und der Betreiber des MR-Scanners. Beispielsweise kann ein solcher Abbruch zu vergeblichen invasiven Maßnahmen führen bei gleichzeitiger Unmöglichkeit, die MR-Messung unmittelbar anschließend zu wiederholen, z.B. bei Kontrastmittelgabe wegen des im Patientengewebe aufgenommenen Kontrastmittels.

Als eine mögliche Aufgabe des vorgeschlagenen Verfahrens kann insbesondere angesehen werden, solche Messabbrüche möglichst zu vermeiden. Die Aufgabe wird durch die Merkmale der unabhängigen Ansprüche gelöst. Vorteilhafte Ausgestaltungen sind in den abhängigen Ansprüchen beschrieben.

Demnach wird ein Verfahren zur Ermittlung eines eine sicherheitsrelevante Größe beschreibenden Simulationswertes für eine MR-Messung vorgeschlagen. Dabei wird eine MR-Pulssequenz bereitgestellt, die ausgebildet ist, anhand ihrer eine MR-Messung eines Patienten mit einem MR-Scanner durchzuführen, wobei die MR-Pulssequenz eine zeitliche Abfolge von mehreren HF-Pulsen und mehreren Gradientenpulsen umfasst. Zudem wird zumindest ein Patientenwert bereitgestellt, der eine Eigenschaft des Patienten beschreibt. Im Fall von mehreren Patientenwerten kann beispielsweise jeder dieser Patientenwerte jeweils eine Eigenschaft beschreiben. Eine solche Eigenschaft kann beispielsweise das Gewicht, die Größe, das Alter oder das Geschlecht des Patienten sein. Ferner kann eine solche Eigenschaft räumliche Ausmaße der im Untersuchungsraum befindlichen Anatomie des Patienten (z.B. vorab durch eine 3D-Kamera erfasst) sowie eine relative Verteilung von Muskeln und Fett im Untersuchungsraum betreffen. Eine weiterer möglicher Patientenwert kann beispielsweise auch die Frage betreffen, ob der Patient ein Träger eines Implantats ist. Ferner kann der zumindest eine Patientenwert die Eigenschaft der Lagerung des Patienten in dem MR-Scanner beschreiben, z.B. wie der Patienten im Untersuchungsraum positioniert ist, insbesondere ob der Patienten mit dem Kopf oder dem Fuß voran im Untersuchungsraum positioniert ist.

Anhand der MR-Pulssequenz und des zumindest einen Patientenwertes wird durch eine Recheneinheit zumindest ein Simulationswert ermittelt. Der zumindest eine Simulationswert beschreibt eine sicherheitsrelevante Größe bei einer Durchführung einer MR-Messung anhand der MR-Pulssequenz. Bei der Ermittlung des zumindest einen Simulationswertes werden spezifische Eigenschaften der HF-Pulse, insbesondere aller HF-Pulse, und der Gradientenpulse, insbesondere aller Gradientenpulse, der MR-Pulssequenz sowie deren zeitliche Abfolge berücksichtigt. Ferner wird der zumindest eine Simulationswert bereitgestellt.

Das Bereitstellen der MR-Pulssequenz kann beispielsweise durch eine erste Schnittstelle erfolgen. Das Bereitstellen des zumindest einen Simulationswertes kann beispielsweise durch eine zweite Schnittstelle erfolgen. Das Bereitstellen der MR-Pulssequenz und/oder des zumindest einen Simulationswertes kann beispielsweise in Form eines Datensatzes erfolgen.

Die bereitgestellte MR-Pulssequenz ist insbesondere ausgebildet, anhand ihrer eine vollständige MR-Messung eines Patienten mit einem MR-Scanner durchzuführen. Die MR-Pulssequenz umfasst und/oder beschreibt vorzugsweise alle HF-Pulse und alle Gradientenpulse, die während der MR-Messung eingestrahlt bzw. geschalten werden. Sie umfasst vorzugsweise alle Angaben, die notwendig sind, um die gewünschten HF-Pulse und alle Gradientenpulse der MR-Pulssequenz zu definieren, die während der MR-Messung angewendet werden. Die MR-Messung ist vorzugsweise geeignet, um dabei MR-Signale aufzunehmen, aus denen zumindest eine, insbesondere zweidimensionale, MR-Abbildung rekonstruiert werden kann.

Die sicherheitsrelevante Größe kann insbesondere die Sicherheit des Patienten und/oder die Sicherheit des MR-Scanners betreffen. Vorteilhafterweise kann aus der sicherheitsrelevanten Größe auf ein Risiko geschlossen werden, ob der Patient und/oder der MR-Scanner Schaden nehmen würde, falls der MR-Scanner die MR-Sequenz anwenden bzw. ausspielen würde.

Die bei der Ermittlung des zumindest einen Simulationswertes zu berücksichtigenden spezifischen Eigenschaften können insbesondere eine Form und/oder eine Dauer und/oder eine Amplitude der HF-Pulse und/oder der Gradientenpulse umfassen. Vorzugsweise umfasst die Ermittlung des zumindest einen Simulationswertes ein Ausrollen der MR-Pulssequenz. Vorzugsweise umfasst das Ausrollen der MR-Pulssequenz eine vollständige Simulation der MR-Pulssequenz, insbesondere unter Berücksichtigung aller HF-Pulse und aller Gradientenpulse. Vorzugsweise greift man beim Ausrollen auf Rohinformationen der MR-Pulssequenz zurück und nicht etwa auf irgendwelche bereits verdichtete und/oder abgeleitete Größen der MR-Pulssequenz. Insbesondere wird beim Ausrollen die spezielle Form und/oder eine Dauer und/oder eine Amplitude der HF-Pulse und/oder der Gradientenpulse eines jeden Pulses sowie die zeitlichen Abstände zwischen den Pulsen betrachtet. Vorzugsweise erfolgt diese Betrachtung nicht nur für einen Teilabschnitt der MR-Pulssequenz, sondern für die ganze MR-Pulssequenz.

Vorteilhafterweise kann durch diese Art der Ermittlung des zumindest einen Simulationswertes, insbesondere durch das Ausrollen der MR-Pulssequenz, erreicht werden, dass etwaige speziell an den jeweiligen MR-Pulssequenztyp angepasste Methoden für eine schnelle Vorab-Ermittlung einer Patientenbelastung durch HF-Pulse und/oder Gradientenpulse nicht mehr entwickelt werden müssen, wie es im Stand der Technik üblicherweise der Fall ist. Oftmals werden solche Methoden im Wesentlichen unabhängig von der tatsächlichen MR-Sequenz entwickelt, es wird also nicht zwingend auf die gleichen Daten zugegriffen. Vielmehr obliegt es dem Entwickler der MR-Pulssequenz, eine möglichst sinnvolle "worst case"-Abschätzung zu treffen. Im ungünstigsten Falle führt eine zu wenig restriktiv gewählte Abschätzung zu einem Messabbruch; auf der anderen Seite wird durch eine zu konservative Abschätzung die verfügbare Leistung des MR-Systems nicht abgerufen, was zu unnötig langer Messzeit und/oder reduzierter Bildqualität führen kann. Vorteilhafterweise können derartige Nachteile des Stands der Technik mit Hilfe des vorgeschlagenen Verfahrens überwunden werden.

Vorteilhafterweise wird der zumindest eine Simulationswert in Echtzeit ermittelt und bereitgestellt. Unter "Echtzeit" ist hier vorzugsweise eine Zeitdauer zu verstehen, die kurz genug ist, damit der Ablauf der MR-Messung durch die Ermittlung des zumindest einen Simulationswertes nicht, insbesondere für den Patienten und/oder ein Bedienpersonal merklich, verlängert und/oder behindert wird. Vorteilhafterweise läuft die Ermittlung des zumindest eine Simulationswerts im Hintergrund ab.

Vorzugsweise beschreibt der zumindest eine Simulationswert eine spezifische Absorptionsrate (SAR) und/oder eine Gradientenstimulation.

Die SAR beschreibt üblicherweise die pro Zeiteinheit und pro Patientenmasse absorbierte hochfrequente Energie durch Einstrahlung der HF-Pulse. Die Absorption der HF-Energie kann zur Erwärmung des Körpergewebes des Patienten führen. Die Energieabsorption ist eine wichtige Größe für die Festlegung von Sicherheitsgrenzwerten. Bei unzulässig hoher lokaler Konzentration von HF-Energie können HF-Verbrennungen auftreten (lokale SAR). Bei gleichmäßiger Verteilung der HF-Energie über den ganzen Körper ist die Belastung der Thermoregulation bzw. des Herzkreislaufsystems des Patienten maßgeblich (Ganzkörper-SAR). Die SAR kann beispielsweise durch HF-Pulse mit niedriger Energie, kleinere Flipwinkel, kürzere Repetitionszeit (TR) und/oder Messung weniger Schichten erreicht werden.

Die Gradientenstimulation kann insbesondere eine Stimulation von Nerven des Patienten umfassen. Insbesondere kann die Gradientenstimulation eine periphere Nervenstimulation (PNS) umfassen. Durch zeitlich veränderliche Magnetfelder, können im Körper des Patienten elektrische Ströme induziert und Nerven oder Muskeln stimulieren werden. Diese Stimulation kann vom Patienten als unangenehm empfunden werden.

Vorzugsweise umfasst das Verfahren zudem ein Vergleichen des zumindest einen Simulationswertes mit einem vorgegebenen Grenzwert, und, falls der zumindest eine Simulationswert den vorgegebenen Grenzwert nicht überschreitet, Durchführen einer MR-Messung des Patienten anhand der MR-Pulssequenz mit dem MR-Scanner. Vorteilhafterweise wird dadurch geprüft, ob durch eine Durchführung der MR-Messung gemäß der MR-Sequenz eine Gefährdung des Patienten und/oder des MR-Scanners auftreten würde und nur dann, wenn dies nicht der Fall ist, würde die MR-Messung gemäß der MR-Sequenz durchgeführt werden.

Eine Ausführungsform des Verfahrens sieht vor, dass die Ermittlung des zumindest einen Simulationswertes durch eine nicht-lokale Recheneinheit durchgeführt wird.

Die nicht-lokale Recheneinheit befindet vorzugsweise an einem anderen Ort als der MR-Scanner. Die nicht-lokale Recheneinheit befindet sich beispielsweise nicht im selben Raum und/oder nicht in einem angrenzenden Raum und/oder nicht im selben Gebäude wie der MR-Scanner. Vorzugsweise basiert die nicht-lokale Recheneinheit auf einer IT-Infrastruktur, die über ein Rechnernetz zur Verfügung gestellt wird, ohne dass die IT-Infrastruktur auf einem lokalen Rechner des MR-Scanners installiert ist. Vorzugsweise basiert die nicht-lokale Recheneinheit auf Cloud Computing und/oder einer IT-Infrastruktur, die beispielsweise über das Internet verfügbar gemacht wird.

Vorteilhafterweise umfasst die nicht-lokale Recheneinheit Hochleistungsrechner, die ausgebildet sind, den zumindest eines Simulationswert in Echtzeit zu ermitteln.

Eine weitere Ausführungsform des Verfahrens sieht vor, dass die Recheneinheit eine Datenbank umfasst, wobei die Datenbank Beschreibungen mehrerer MR-Pulssequenztypen umfasst, wobei der Recheneinheit zumindest eine MR-Pulssequenztyp-ID bereitgestellt wird, wobei die zumindest eine MR-Pulssequenztyp-ID einer der mehreren MR-Pulssequenztypen zugeordnet ist, wobei der Recheneinheit zumindest ein MR-Pulssequenzparameter bereitgestellt wird, wobei durch die Recheneinheit die MR-Pulssequenz anhand der zumindest einen MR-Pulssequenztyp-ID und dem zumindest einen MR-Pulssequenzparameter ermittelt wird.

Vorteilhafterweise kann durch eine Vorhaltung und/oder Speicherung der mehreren MR-Pulssequenztypen in der Datenbank erreicht werden, dass nur der zumindest eine MR-Pulssequenzparameter an die Recheneinheit übermittelt werden muss, aber nicht die (gesamte) MR-Pulssequenz.

Ein MR-Pulssequenztyp kann insbesondere eine typspezifische Struktur und/oder ein typspezifisches Muster, insbesondere an HF-Pulsen und/oder Gradientenpulsen, aufweisen. Eine solche Struktur und/oder ein solches Muster kann beispielsweise eine Anordnung miteinander wechselwirkender und/oder verbundener Elemente aufweisen. Solche Elemente können insbesondere HF-Pulse und/oder Gradientenpulse sein.

Vorzugsweise sind die MR-Pulssequenztypen parametrierbar, d.h. durch Angabe des zumindest ein MR-Pulssequenzparameters kann aus einem MR-Pulssequenztyp eine, insbesondere vollständig definierte, MR-Pulssequenz abgeleitet werden, die insbesondere eindeutig eine Abfolge von HF-Pulsen und/oder Gradientenpulsen beschreibt. Insbesondere kann ein MR-Pulssequenztyp ein Gerüst vorgeben, das durch das Bereitstellen des zumindest einen MR-Pulssequenzparameters ausgefüllt werden kann. Ein MR-Pulssequenzparameter kann beispielsweise eine Anzahl an Wiederholungen eines Sequenzabschnitts und/oder eine Flipwinkel etc. umfassen.

Vorzugsweise kann ein MR-Pulssequenztyp einen oder MR-Pulssequenzabschnitte beschreiben, z.B. bei einer Diffusionssequenz bildet jede unterschiedliche Diffusionskodierung einen Unterabschnitt, die Fettsättigung und das Auslesemodul. Die Unterabschnitte können beispielsweise getrennt voneinander parametriert werden.

Eine MR-Pulssequenztyp-ID kann beispielsweise ein Name und/oder eine Nummer sein, mit der ein MR-Pulssequenztyp bezeichnet wird.

Eine weitere Ausführungsform des Verfahrens sieht vor, dass die Recheneinheit eine Datenbank umfasst, wobei die Datenbank zumindest einen vorberechneten Hilfswert umfasst, wobei die Ermittlung zumindest eines Simulationswertes mit Hilfe des zumindest einen Hilfswert erfolgt. Insbesondere ist dem zumindest einem Hilfswert eine Variation von Patientenwerten und/oder MR-Pulssequenzparametern zugeordnet.

Beispielsweise kann die Datenbank zur Ermittlung zumindest eines Simulationswertes, insbesondere einer optimalen SAR-Vorhersage, für zumindest einen MR-Pulssequenztyp zumindest einen vorberechneten Hilfswert umfassen. Beispielsweise können als Hilfswerte Limitierungen für Variationen von Patienten- und Sequenzparametern bereits vorab berechnet und abgespeichert werden.

Vorzugsweise ist der zumindest eine Hilfswert einem Abschnitt der MR-Pulssequenz, also einem MR-Pulssequenzabschnitt zugeordnet. Vorteilhafterweise kann die Ermittlung des zumindest einen Simulationswertes abschnittsweise für die jeweiligen MR-Pulssequenzabschnitte erfolgen.

Der zumindest eine Hilfswert kann insbesondere auf einer Modellierung zumindest eines MR-Pulssequenztyps für zumindest einen Patientenwert und/oder für zumindest einen MR-Pulssequenzparameter basieren. Der zumindest eine Hilfswert kann insbesondere eine Variation einer Messzeit zur Durchführung der MR-Messung berücksichtigen. Der zumindest eine Hilfswert kann insbesondere eine Modellierung des Patienten, eine räumliche Messabdeckung durch aufzunehmende MR-Signale und/oder einen Umfang aufzunehmender MR-Signale betreffen. Beispielsweise können ein oder mehrere MR-Pulssequenztypen für eine Reihe an Patientenparametern, wie z.B. Gewicht und/oder Körpergröße, modelliert und das Ergebnis der Modellierung als Hilfswerte abgespeichert worden sein. Beispielsweise kann für eine optimale Stimulationsvorhersage eine Kippung einer Schichtorientierung für eine Anzahl an Winkeln erfolgt sein. Weiterhin kann beispielsweise ein Einfluss einer Messzeitverlängerung, z.B. durch Erhöhung einer Anzahl von Mittelungen und/oder einer Matrix-Größe, vorab geprüft werden. Vorteilhafterweise ermöglicht dieses Vorgehen insbesondere eine Echtzeitvorhersage von Einstellungsänderungen auf die Lauffähigkeit einer MR-Sequenzen während einer Editierung einer der MR-Sequenz.

Der zumindest eine Hilfswert kann insbesondere einen MR-Pulssequenzabschnitt betreffen. Insbesondere kann zumindest ein Hilfswert für einen MR-Pulssequenzabschnitt berechnet werden. Vorzugsweise können MR-Pulssequenzabschnitte z.B. mittels zusätzlicher Parametrisierung, die beeinflussende Werte aus einem vorangehenden MR-Pulssequenzabschnitt beschreiben (z.B. bereits angefallene SAR, aktueller Stimulationswert etc.) schnell neu berechnet und/oder kombiniert werden. Insbesondere kann eine MR-Pulssequenz aus bekannten Blöcken zusammengesetzt werden.

Eine weitere Ausführungsform des Verfahrens sieht vor, dass zumindest ein Justagewert bereitgestellt wird, z.B. durch eine dritte Schnittstelle. Dabei erfolgt die Ermittlung des zumindest einen Simulationswertes durch die Recheneinheit auch anhand des zumindest einen Justagewerts.

Der zumindest eine Justagewert kann insbesondere eine, insbesondere temporäre, Eigenschaft und/oder einen Betriebsparameter des MR-Scanners beschreiben. Diese Eigenschaft und/oder dieser Betriebsparameter kann insbesondere eine HF-Sendespannung, insbesondere eine maximale HF-Amplitude, und/oder einen patientenabhängigen Skalierungsfaktor, der eine Umrechnung von Flipwinkel in HF-Sendespannung erlaubt, und/oder ein Gradientenoffset, das ein Ausgleich von äußeren oder Patientenspezifischer Magnetfeldabweichungen ermöglicht, betreffen.

Vorteilhafterweise kann die Ermittlung des zumindest einen Simulationswertes mit Hilfe des zumindest einen Justagewerts noch genauer erfolgen.

Eine weitere Ausführungsform des Verfahrens sieht vor, dass der Recheneinheit eine Protokoll-Queue, insbesondere einen Protokollsatz, mit mehreren MR-Pulssequenzen bereitgestellt wird, wobei für jede der mehreren MR-Pulssequenzen zumindest ein Simulationswert durch die Recheneinheit ermittelt werden. Vorzugsweise umfasst eine solche Protokoll-Queue alle MR-Pulssequenzen, die im Laufe einer MR-Untersuchung eines Patienten gemessen werden. Beispielsweise wird zunächst eine Localizer-Messung durchgeführt, worauf, insbesondere automatisch, eine Messplanung folgt, woraus die Protokoll-Queue resultiert. Vorzugsweise wird nicht erst dann, wenn eine MR-Sequenz an der Reihe ist, sondern bereits vorher die MR-Sequenz ausgerollt und/oder überprüft.

Vorzugsweise umfasst eine Protokoll-Queue eine zeitliche Abfolge von mehreren MR-Pulssequenzen. Vorzugsweise beschreibt jede dieser MR-Pulssequenzen jeweils eine MR-Messung. Insbesondere kann die Ermittlung des zumindest einen Simulationswertes (und auch ein mögliches Vergleichen des zumindest einen Simulationswertes mit einem vorgegebenen Grenzwert) für auf eine aktuelle MR-Messung nachfolgende und/oder schon geplante MR-Messungen in der Protokoll-Queue erfolgen. Vorteilhafterweise können so insbesondere mögliche Überschreitungen frühzeitig erkannt werden, und/oder eine Auflösung etwaiger Konflikte ist rechtzeitig möglich.

Eine weitere Ausführungsform des Verfahrens sieht vor, dass anhand des Simulationswert die MR-Pulssequenz, insbesondere zumindest ein MR-Pulssequenzparameter, optimiert wird. Diese Optimierung kann insbesondere mittels eines neuronalen Netzes erfolgen. Vorteilhafterweise könnte eine solche Optimierung durch eine nicht-lokale Recheneinheit durchgeführt werden. Insbesondere kann auf leistungsfähigen Rechnern in der Cloud eine komplexere Optimierung von einstellbaren MR-Pulssequenzparametern erfolgen.

Es ist denkbar, dass der Optimierung automatisch, insbesondere ohne Zutun durch ein Bedienpersonal des MR-Scanners erfolgt. Es ist aber auch denkbar, dass einem Bedienpersonal des MR-Scanners ein Vorschlag, insbesondere im Rahmen einer Vorschau, unterbreitet wird, gemäß dem zumindest ein MR-Pulssequenzparameter der MR-Pulssequenz angepasst werden kann. Das Bedienpersonal kann den Vorschlag beispielsweise ablehnen, unverändert annehmen oder Änderungen an dem Vorschlag vornehmen.

Ferner wird eine Recheneinheit zur Ermittlung zumindest eines Simulationswertes vorgeschlagen, die ausgebildet ist, anhand einer MR-Pulssequenz und zumindest eines Patientenwertes den zumindest einen Simulationswert zu ermitteln, wobei die MR-Pulssequenz eine zeitliche Abfolge von mehreren HF-Pulsen und mehreren Gradientenpulsen umfasst, wobei der zumindest eine Patientenwert eine Eigenschaft des Patienten beschreibt, wobei der zumindest eine Simulationswert eine sicherheitsrelevante Größe bei einer Durchführung einer MR-Messung anhand der MR-Pulssequenz beschreibt, wobei bei die Recheneinheit ferner ausgebildet ist, bei einer Ermittlung des zumindest einen Simulationswertes spezifische Eigenschaften, insbesondere Form und/oder Dauer und/oder Amplitude, aller HF-Pulse und aller Gradientenpulse der MR-Pulssequenz sowie deren zeitliche Abfolge zu berücksichtigen.

Die Vorteile der vorgeschlagenen Recheneinheit zur Ermittlung des zumindest einen Simulationswertes entsprechen im Wesentlichen den Vorteilen eines Verfahrens zur Ermittlung eines eine sicherheitsrelevante Größe beschreibenden Simulationswertes für eine MR-Messung, wie es vorab im Detail beschrieben wird. Hierbei erwähnte Merkmale, Vorteile oder alternative Ausführungsformen können ebenso auch auf die anderen beanspruchten Gegenstände übertragen werden und umgekehrt.

Ferner wird ein MR-Scanner mit einer vorab beschriebenen Recheneinheit vorgeschlagen.

Ferner wird ein Computerprogrammprodukt vorgeschlagen, das ein Programm umfasst und direkt in einen Speicher einer Recheneinheit zur Ermittlung zumindest eines Simulationswertes ladbar ist und Programmmittel, z.B. Bibliotheken und Hilfsfunktionen, aufweist, um ein erfindungsgemäßes Verfahren auszuführen, wenn das Computerprogrammprodukt in der Recheneinheit ausgeführt wird. Das Computerprogrammprogrammprodukt kann dabei eine Software mit einen Quellcode, der noch kompiliert und gebunden oder der nur interpretiert werden muss, oder einen ausführbaren Softwarecode umfassen, der zur Ausführung nur noch in die Systemsteuereinheit zu laden ist. Durch das Computerprogrammprodukt kann das erfindungsgemäße Verfahren schnell, identisch wiederholbar und robust ausgeführt werden. Das Computerprogrammprodukt ist so konfiguriert, dass es mittels der Recheneinheit entsprechende Verfahrensschritte ausführen kann. Die Recheneinheit weist dabei vorteilhafterweise die Voraussetzungen wie beispielsweise einen entsprechenden Arbeitsspeicher, eine entsprechende Grafikkarte oder eine entsprechende Logikeinheit auf, so dass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt. Insbesondere kann das Computerprogrammprodukt in einen Prozessor einer lokalen Systemsteuereinheit geladen werden kann, der mit einem MR-Scanner direkt verbunden oder als Teil des MR-Scanners ausgebildet sein kann.

Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Recheneinheit ein erfindungsgemäßes Verfahren durchführen. Beispiele für elektronische lesbare Datenträger sind eine DVD, ein Magnetband oder einen USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: einen MR-Scanner und eine nicht-lokale Recheneinheit,
- Fig. 2: ein Verfahren zur Ermittlung eines eine sicherheits-relevante Größe beschreibenden Simulationswertes für eine MR-Messung,
- Fig. 3: mögliche Informationsströme zwischen einer Recheneinheit und einem MR-Scanner zur Durchführung eines Verfahrens zur Ermittlung eines eine sicherheitsrelevante Größe beschreibenden Simulationswertes für eine MR-Messung,
- Fig. 4: eine MR-Pulssequenz mit mehreren HF-Pulsen und Gradientenpulsen.

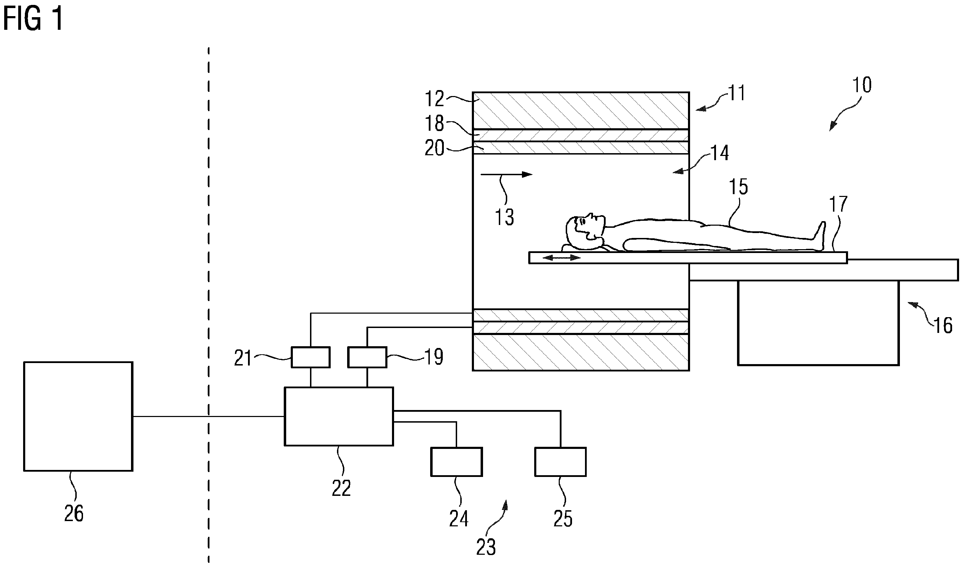

In Fig. 1 ist ein MR-Scanner 10 und eine nicht-lokale Recheneinheit 26 schematisch dargestellt. Der MR-Scanner 10 umfasst eine Magneteinheit 11, die einen Hauptmagneten 12 zu einem Erzeugen eines starken und insbesondere zeitlich konstanten Hauptmagnetfelds 13 aufweist. Zudem umfasst der MR-Scanner 10 einen Patientenaufnahmebereich 14 zu einer Aufnahme eines Patienten 15. Der Patientenaufnahmebereich 14 ist im vorliegenden Ausführungsbeispiel zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 denkbar. Der Patient 15 kann mittels einer Patientenlagerungsvorrichtung 16 des MR-Scanners 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradientenpulsen, kurz: Gradientenpulsen, auf. Die Gradientenpulse werden während einer MR-Messung insbesondere für eine Ortskodierung verwendet. Die Gradientenspuleneinheit 18 wird mittels einer Gradientensteuereinheit 19 des MR-Scanners 10 gesteuert. Die Magneteinheit 11 umfasst weiterhin eine Hochfrequenzantenneneinheit 20, welche im vorliegenden Ausführungsbeispiel als eine fest in den MR-Scanner 10 integrierte Körperspule ausgebildet ist. Die Hochfrequenzantenneneinheit 20 wird von einer Hochfrequenzantennensteuereinheit 21 des MR-Scanners 10 gesteuert und strahlt hochfrequente (HF) Pulse in einen Untersuchungsraum ein, der im Wesentlichen von einem Patientenaufnahmebereich 14 des MR-Scanners 10 gebildet ist. Dadurch stellt sich dem von dem Hauptmagneten 12 erzeugten Hauptmagnetfeld 13 eine Anregung von Atomkernen ein. Durch Relaxation der angeregten Atomkerne werden Magnetresonanzsignale erzeugt. Die Hochfrequenzantenneneinheit 20 ist zum Empfang der Magnetresonanzsignale ausgebildet.

Zu einer Steuerung des Hauptmagneten 12, der Gradientensteuereinheit 19 und zur Steuerung der Hochfrequenzantennensteuereinheit 21 weist der MR-Scanner 10 eine Systemsteuereinheit 22 auf. Die Systemsteuereinheit 22 steuert die Magnetresonanzvorrichtung 10, wie insbesondere das Durchführen einer MR-Pulssequenz. Zudem umfasst die Systemsteuereinheit 22 eine nicht näher dargestellte Auswerteeinheit zu einer Auswertung der MR-Signale, die während einer MR-Messung erfasst werden. Des Weiteren umfasst der MR-Scanner 10 eine Benutzerschnittstelle 23, die mit der Systemsteuereinheit 22 verbunden ist. Steuerinformationen wie beispielsweise MR-Pulssequenzparameter, sowie rekonstruierte MR-Abbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter, insbesondere MR-Pulssequenzparameter, während eines Messvorgangs von dem medizinischen Bedienpersonal eingegeben werden können.

Die Systemsteuereinheit 22 des MR-Scanners ist mit einer Recheneinheit 26 verbunden. Im vorliegenden Beispiel handelt es sich bei der Recheneinheit 26 um eine nicht-lokale Recheneinheit. Die nicht-lokale Recheneinheit 26 ist getrennt von dem MR-Scanner 10 und ist über eine Übertragungsleitung mit dem MR-Scanner 10 verbunden. Es ist aber auch denkbar, dass die Recheneinheit 26 eine lokale Recheneinheit ist. Insbesondere ist denkbar, dass die Recheneinheit 26 ein Teil der Systemsteuereinheit 22 des MR-Scanners 10 ist.

Die Recheneinheit 26 ist ausgebildet, anhand einer MR-Pulssequenz und zumindest eines Patientenwertes zumindest einen Simulationswert zu ermitteln und der Systemsteuereinheit 22 bereitzustellen. Ist es ferner denkbar, dass die Recheneinheit 26 ausgebildet ist, der Systemsteuereinheit 22 eine optimierte MR-Pulssequenz, insbesondere MR-Pulssequenzparameter einer optimierten MR-Pulssequenz, bereitzustellen.

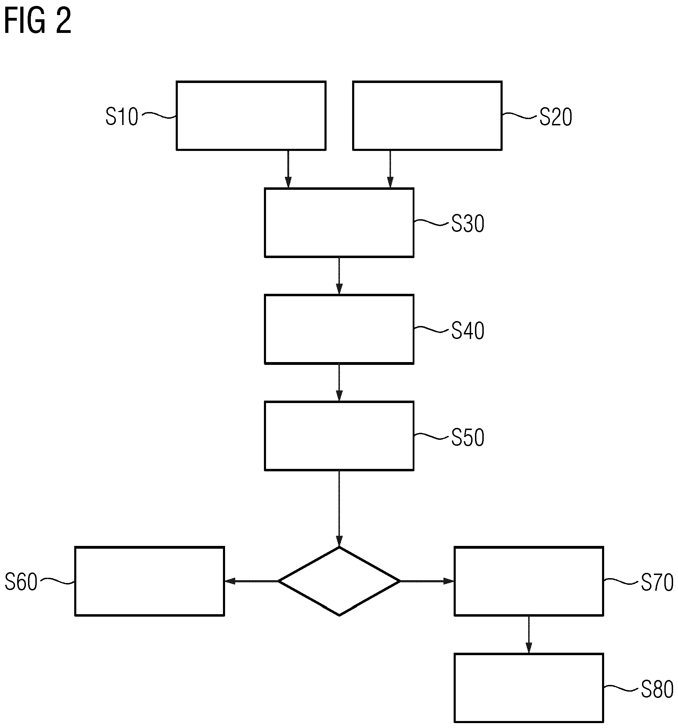

Ein entsprechendes Verfahren zur Ermittlung eines eine sicherheitsrelevante Größe beschreibenden Simulationswertes für eine MR-Messung ist in Fig. 2 dargestellt. In S10 wird der Recheneinheit 26 durch die Systemsteuereinheit 22 eine MR-Pulssequenz bereitgestellt, die ausgebildet ist, anhand ihrer eine MR-Messung eines Patienten 15 mit einem MR-Scanner 10 durchzuführen. Dabei umfasst die MR-Pulssequenz eine zeitliche Abfolge von mehreren HF-Pulsen, die mit Hilfe der Hochfrequenzantenneneinheit 20 des MR-Scanners 10 ausgegeben werden können, und mehreren Gradientenpulsen, die mit Hilfe der Gradientenspuleneinheit 18 des MR-Scanners 10 ausgegeben werden können.

In S20 wird der Recheneinheit 26 durch die Systemsteuereinheit 22 zumindest ein Patientenwert bereitgestellt, wobei der zumindest eine Patientenwert eine Eigenschaft des Patienten 15 beschreibt.

In S30 wird zumindest ein Simulationswert durch die Recheneinheit 26 anhand der MR-Pulssequenz und des zumindest einen Patientenwertes ermittelt. Dabei beschreibt der zumindest eine Simulationswert eine sicherheitsrelevante Größe bei einer Durchführung einer MR-Messung anhand der MR-Pulssequenz, wie beispielsweise eine spezifische Absorptionsrate und/oder eine Gradientenstimulation, insbesondere eine Nervenstimulation. Bei der Ermittlung des zumindest einen Simulationswertes in S30 werden spezifische Eigenschaften, insbesondere Form und/oder Dauer und/oder Amplitude, der HF-Pulse, insbesondere aller HF-Pulse, und der Gradientenpulse, insbesondere aller Gradientenpulse, der MR-Pulssequenz sowie deren zeitliche Abfolge berücksichtigt. In S40 wird der zumindest eine Simulationswert von der Recheneinheit 26 an die Systemsteuereinheit 22 bereitgestellt.

In diesem Beispiel wird in S50 der zumindest eine Simulationswert mit einem vorgegebenen Grenzwert verglichen. Dieser Vergleich kann beispielsweise durch die Recheneinheit 26 und/oder durch die Systemsteuereinheit 22 erfolgen.

Falls der zumindest eine Simulationswert den vorgegebenen Grenzwert nicht überschreitet, wird in S60 eine MR-Messung des Patienten 15 anhand der MR-Pulssequenz mit dem MR-Scanner 10 durchgeführt.

Es kann insbesondere vorgesehen sein, dass in S70 anhand des Simulationswertes die MR-Pulssequenz, insbesondere mittels eines neuronalen Netzes, optimiert wird. Dies erfolgt vorteilhafterweise dann, wenn der zumindest eine Simulationswert den vorgegebenen Grenzwert überschreitet. Die optimierte MR-Pulssequenz kann in S80 der Systemsteuereinheit 22 bereitgestellt werden.

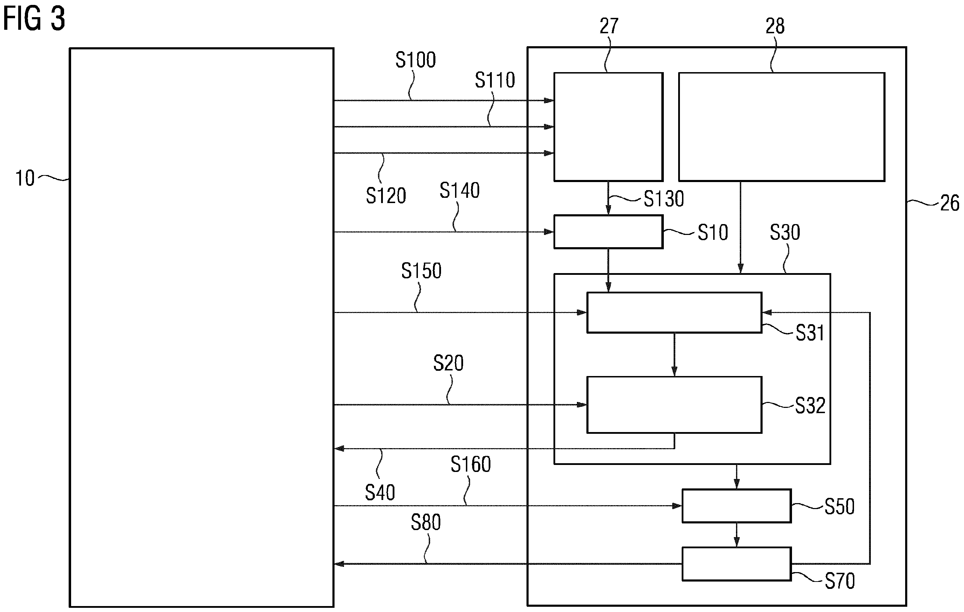

Anhand von Fig. 3 werden weitere mögliche Varianten bzw. Details illustriert. Die Recheneinheit 26 umfasst hier insbesondere eine Datenbank 27, die Beschreibungen mehrerer MR-Pulssequenztypen umfasst. In die Datenbank könnten beispielsweise in S100 weitere Beschreibungen von MR-Pulssequenztypen importiert werden. Dabei kann es sich beispielsweise um MR-Pulssequenztypen handeln, die von beliebigen Dritten, d.h. nicht vom Hersteller des MR-Scanners 10, entwickelt wurden.

In S110 stellt der MR-Scanner 10 der Recheneinheit eine MR-Pulssequenztyp-ID bereit, die einer der mehreren MR-Pulssequenztypen, die in der Datenbank 27 hinterlegt ist, zugeordnet werden kann. Außerdem stellt der MR-Scanner in S120 der Recheneinheit 26 mehrere MR-Pulssequenzparameter bereit. In S130 kann durch die Recheneinheit 26 die MR-Pulssequenz, für die zumindest ein Simulationswert ermittelt werden soll, anhand der MR-Pulssequenztyp-ID und mehreren MR-Pulssequenzparameter ermittelt werden.

Es ist aber auch denkbar, dass in S140 die MR-Pulssequenz ohne Rückgriff auf eine Datenbank 27 an die Recheneinheit 26 übermittelt wird. Durch S130 und/oder S140 kann das Bereitstellen der MR-Pulssequenz in S10 erfolgen.

Die Recheneinheit 26 kann ferner eine Datenbank 28 umfassen, die zumindest einen vorberechneten Hilfswert umfasst. Dem zumindest einen Hilfswert ist beispielsweise eine Variation von Patientenwerten und/oder MR-Pulssequenzparametern zugeordnet. Der zumindest eine Hilfswert kann beispielsweise auf einer Modellierung zumindest einen MR-Pulssequenztyps für zumindest einen Patientenwert und/oder für zumindest einen MR-Pulssequenzparameter basieren. Ferner kann der zumindest eine Hilfswert eine Variation einer Messzeit zur Durchführung der MR-Messung berücksichtigen. Die Ermittlung des zumindest eines Simulationswertes in S30 kann mit Hilfe des zumindest einen Hilfswerts erfolgen.

In S150 werden der Recheneinheit zumindest ein Justagewert bereitgestellt, der für die Ermittlung des zumindest einen Simulationswertes durch die Recheneinheit in S31 und/oder S32 verwendet wird.

Anhand der MR-Pulssequenz und wird die MR-Pulssequenz in S31 ausgerollt. Hierbei werden spezifische Eigenschaften, insbesondere Form und/oder Dauer und/oder Amplitude, aller HF-Pulse und aller Gradientenpulse der MR-Pulssequenz sowie deren zeitliche Abfolge berücksichtigt.

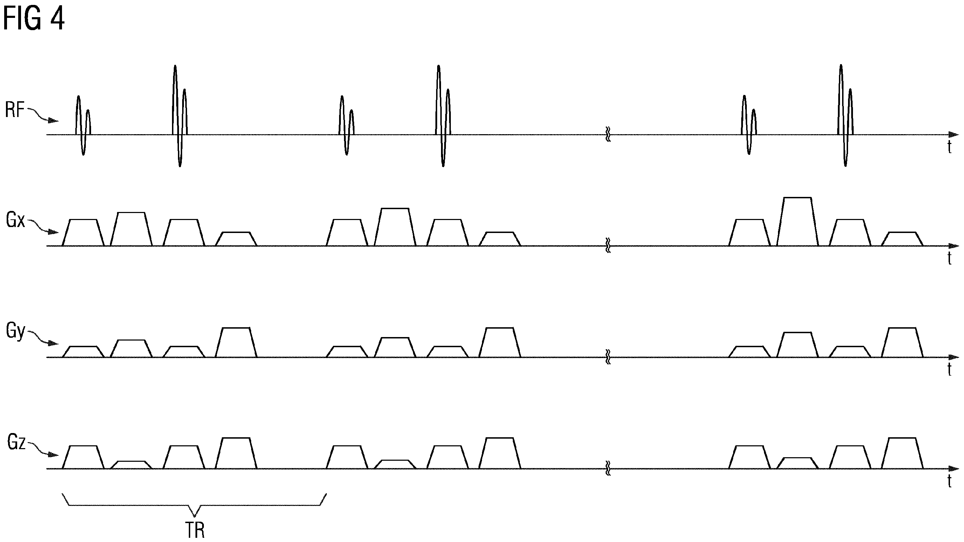

Anhand von Fig. 4 soll das Ausrollen der MR-Pulssequenz in S31 illustriert werden, in der mehrere Achsen in Abhängigkeit zur Zeit t dargestellt sind. Die MR-Pulssequenz lässt sich beschreiben durch mehrere HF-Pulse die entlang der Achse RF dargestellt sind. In bestimmten zeitlichen Abständen können diese Pulse durch die Hochfrequenzantenneneinheit 20 des MR-Scanners 10 ausgegeben werden. Ferner sind entlang der Achsen Gx, Gy und Gz mehrere Gradientenpulse dargestellt. Dabei repräsentiert Gx eine Gradientenspule der Gradientenspuleneinheit 18, die entlang einer x-Richtung einen Magnetfeldgradienten erzeugen kann; Gy repräsentiert eine Gradientenspule der Gradientenspuleneinheit 18, die entlang einer y-Richtung einen Magnetfeldgradienten erzeugen kann; Gz repräsentiert eine Gradientenspule der Gradientenspuleneinheit 18, die entlang einer γ-Richtung einen Magnetfeldgradienten erzeugen kann. Vorzugsweise bilden x, y und z ein orthogonales Koordinatensystem. Bestimmte Muster wiederholen sich nach bestimmten Repetitionszeiten TR, wobei beispielsweise jeweils ein Parameter variiert wird. So kann beispielsweise sukzessive eine ganze Schicht des Patienten 15 gemessen werden.

Die HF-Pulse entlang der Achse RF und die Gradientenpulse entlang der Achsen Gx, Gy und Gz weisen jeweils spezifische Eigenschaften auf, insbesondere eine spezifische Form und/oder Dauer und/oder Amplitude. Beim Ausrollen werden vorzugsweise alle diese Pulse mit ihren Eigenschaften betrachtet, also insbesondere ihre Auswirkungen auf den zu ermittelnden zumindest einen Simulationswert berücksichtigt.

Gemäß Fig. 2 wird anhand der in S31 ausgerollten MR-Pulssequenz in S32 der zumindest eine Simulationswert ermittelt. Für diese Ermittlung wird der Recheneinheit in S20 zumindest ein Patientenwert bereitgestellt. Der ermittelte zumindest eine Simulationswert wird in S40 dem MR-Scanner bereitgestellt. Der zumindest eine Simulationswert kann beispielsweise mit durch die Anzeigeeinheit 24 des MR-Scanners angezeigt und/oder durch die Systemsteuereinheit 22 weiterverarbeitet werden.

In S50 kann der zumindest eine Simulationswert mit zumindest einen Grenzwert verglichen werden, der in S160 bereitgestellt wird. Der Vergleich wird in diesem Beispiel durch die Recheneinheit 26 durchgeführt. Es ist aber auch denkbar, dass der Vergleich beispielsweise in der Systemsteuereinheit 22 des MR-Scanners durchgeführt wird.

Gegebenenfalls kann in S70 eine Optimierung der MR-Pulssequenz erfolgen, insbesondere dann, wenn der Vergleich ergibt, dass der Grenzwert nicht eingehalten wird. Die optimierte MR-Pulssequenz kann in S31 nochmals ausgerollt werden, so dass in S32 für die optimierte MR-Pulssequenz ein weiterer Simulationswert ermittelt wird, der wiederum in S50 mit einem Grenzwert verglichen werden kann und ggf. abermals optimiert werden kann. Schließlich kann eine mögliche optimierte Sequenz in S80 dem MR-Scanner 10 bereitgestellt werden.

Wegen eines möglicherweise hohen Rechenaufwands können, die Schritte, die in der Recheneinheit 26 durchgeführt werden, vorzugsweise Cloud-basiert erfolgen. Insbesondere können die Beschreibungen der mehrerer MR-Pulssequenztypen bereits in der Cloud vorliegen, so dass nur noch die eingestellten Protokollparameter in S120 vom MR-Scanner 10 übermittelt werden müssen. Gegebenenfalls können auch Daten für eine aktuelle Mess-Situation (etwa Justageparameter in S150 oder Patientenparameter, wie z.B. Größe, Gewicht, Position und Orientierung, in S20) mit in die Cloud übertragen werden.

Aus der Cloud werden dann die ermittelten Simulationswerte, insbesondere Vorhersagewerte, zurückgeliefert, nachdem dort die Sequenzen auf schnellen Hochleistungsrechnern in S31 komplett ausgerollt wurde.

Eine denkbare Erweiterung besteht darin, die entsprechenden Grenzwerte der zu ermittelnden Größen in S160 mit in die Cloud durchzureichen, um bei Überschreitung eines Limits ggf. möglichst sinnvoll geänderte MR-Pulssequenzparameter für die Sequenz vorzuschlagen. Auf den leistungsfähigen Rechnern in der Cloud könnten komplexere Optimierungen der einstellbaren Protokollparameter erfolgen und/oder ein neuronales Netz zur Verwendung kommen. Mit den geänderten MR-Pulssequenzparametern können beispielsweise auch die zugehörigen zu begrenzenden Größen als Vorschau in S40 zurückgeliefert werden, die beispielsweise mit der Anzeigeeinheit 24 der Benutzerschnittstelle 23 des MR-Scanners 10 angezeigt werden können.

Zur Bereitstellung der Hilfswerte für die Datenbank 28 können beispielsweise Begrenzungen für Variationen von Patienten-und Sequenzparametern bereits vorab berechnet und abgespeichert werden. So kann beispielsweise für eine optimale SAR-Vorhersage jedes Protokoll bereits für eine Reihe an Patientenparametern wie Gewicht und Körpergröße modelliert worden sein und für eine optimale Stimulationsvorhersage kann eine Kippung der Schichtorientierung für eine Anzahl an Winkeln erfolgt sein. Weiterhin kann der Einfluss einer Messzeitverlängerung (z.B. Erhöhung von Mittelungen oder der Matrix-Größe) vorab geprüft werden. Dieses Vorgehen ermöglicht insbesondere eine Echtzeitvorhersage von Einstellungsänderungen auf die Lauffähigkeit von Protokollen während der Protokolleditierung.

In einer bevorzugten Variante erfolgt die Grenzwert-Überprüfung in S50 insbesondere für auf die aktuelle Messung nachfolgende und schon geplante Messungen in der Protokoll-Queue. So können mögliche Überschreitungen frühzeitig erkannt werden und eine Auflösung von Konflikten ist ohne "Last Minute"-Änderungen möglich.

In einer Variante kann auf die Nutzung einer Cloud verzichtet werden. Stattdessen kann beispielsweise auch die Systemsteuereinheit 22 des MR-Scanners die Recheneinheit 26 umfassen, so dass sie lokal am MR-Scanner integriert ist.

In einer weiteren Ausgestaltungsform werden Belastungspausen (z.B. während Messpausen oder über Nacht) vorhandener Recheneinheiten des MR-Scanners, wie z.B. Host- oder MARS-Rechner benutzt, um Modelle für neu importiere MR-Sequenzen im Voraus zu errechnen, so dass diese dann später beim Start der MR-Messung zur Verfügung stehen.

Mit Hilfe des hier vorgeschlagenen Verfahrens bzw. Vorrichtungen ergeben sich vorzugsweise folgende Vorteile: Es können vorteilhafterweise alle Varianten von MR-Pulssequenztypen abgedeckt werden. Ferner können weiteren sonst nicht berücksichtigte Begrenzungen (wie z.B. Duty Cycle-Modelle) einbezogen werden, ohne dass spezielle neue Methoden dafür entwickelt werden müssen. Ferner können dadurch idealerweise Abschaltungen während des Messbetriebs wegen GrenzwertÜberschreitungen vermieden werden. Zudem wird eine konsequente Umsetzung von Sicherheitsarchitekturen ermöglicht, die sich nicht ausschließlich auf eine Online-Abschaltung verlassen, sondern bereits bei der Beauftragung konsequent potentielle Überschreitungen ausschließen; dies ist besonders vorteilhaft angesichts besonderer, möglicherweise lebensbedrohlicher Konsequenzen insbesondere bei aktiven Implantaten wie Herz-Schrittmachern oder auch Deep Brain Stimulators. Ferner können weitergehende Gegenvorschläge für optimierte MR-Pulssequenzen, insbesondere MR-Pulssequenzparameter während des üblichen Arbeitsablaufs zur Verfügung gestellt werden, falls eine aktuell eingestellte MR-Pulssequenz zu einer Überschreitung von Limitierungen führen würden.

Es wird abschließend noch einmal darauf hingewiesen, dass es sich bei den vorhergehend detailliert beschriebenen Verfahren sowie bei der dargestellten Recheneinheit und dem MR-Scanner lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Ermittlung eines eine sicherheitsrelevante Größe beschreibenden Simulationswertes für eine MR-Messung umfassend:
Bereitstellen einer MR-Pulssequenz, die ausgebildet ist, anhand ihrer eine MR-Messung eines Patienten mit einem MR-Scanner durchzuführen,
wobei die MR-Pulssequenz eine zeitliche Abfolge von mehreren HF-Pulsen und mehreren Gradientenpulsen umfasst,
Bereitstellen zumindest eines Patientenwertes,
wobei der zumindest eine Patientenwert eine Eigenschaft des Patienten beschreibt,
Ermittlung zumindest eines Simulationswertes durch eine Recheneinheit anhand der MR-Pulssequenz und des zumindest einen Patientenwertes,
wobei der zumindest eine Simulationswert eine sicherheitsrelevante Größe bei einer Durchführung einer MR-Messung anhand der MR-Pulssequenz beschreibt,
wobei bei der Ermittlung des zumindest einen Simulationswertes spezifische Eigenschaften, insbesondere Form und/oder Dauer und/oder Amplitude, der HF-Pulse und der Gradientenpulse der MR-Pulssequenz sowie deren zeitliche Abfolge berücksichtigt werden,
Bereitstellen des zumindest einen Simulationswertes.

2. Verfahren nach Anspruch 1,
wobei der zumindest eine Simulationswert eine spezifische Absorptionsrate und/oder eine Gradientenstimulation, insbesondere eine Nervenstimulation, beschreibt.

3. Verfahren nach einem der vorangehenden Ansprüche,
wobei das Verfahren zudem umfasst:
Vergleichen des zumindest einen Simulationswertes mit einem vorgegebenen Grenzwert,
und, falls der zumindest eine Simulationswert den vorgegebenen Grenzwert nicht überschreitet, Durchführen einer MR-Messung des Patienten anhand der MR-Pulssequenz mit dem MR-Scanner.

4. Verfahren nach einem der vorangehenden Ansprüche,
wobei die Ermittlung des zumindest einen Simulationswertes durch eine nicht-lokale Recheneinheit durchgeführt wird.

5. Verfahren nach einem der vorangehenden Ansprüche,
wobei die Recheneinheit eine Datenbank umfasst,
wobei die Datenbank Beschreibungen mehrerer MR-Pulssequenztypen umfasst,
wobei der Recheneinheit zumindest eine MR-Pulssequenztyp-ID bereitgestellt wird,
wobei die zumindest eine MR-Pulssequenztyp-ID einer der mehreren MR-Pulssequenztypen zugeordnet ist,
wobei der Recheneinheit zumindest ein MR-Pulssequenzparameter bereitgestellt wird,
wobei durch die Recheneinheit die MR-Pulssequenz anhand der zumindest einen MR-Pulssequenztyp-ID und dem zumindest einen MR-Pulssequenzparameter ermittelt wird.

6. Verfahren nach einem der vorangehenden Ansprüche,
wobei die Recheneinheit eine Datenbank umfasst,
wobei die Datenbank zumindest einen vorberechneten Hilfswert umfasst,
wobei die Ermittlung zumindest eines Simulationswertes mit Hilfe des zumindest einen Hilfswert erfolgt.

7. Verfahren nach Anspruch 6,
wobei dem zumindest eine Hilfswert eine Variation von Patientenwerten und/oder MR-Pulssequenzparametern zugeordnet ist.

8. Verfahren nach Anspruch 6 oder 7,
wobei der zumindest eine Hilfswert auf einer Modellierung zumindest einen MR-Pulssequenztyps für zumindest einen Patientenwert und/oder für zumindest einen MR-Pulssequenzparameter basiert.

9. Verfahren nach einem der Ansprüche 6 bis 8.
wobei der zumindest eine Hilfswert eine Variation einer Messzeit zur Durchführung der MR-Messung berücksichtigt.

10. Verfahren nach einem der vorangehenden Ansprüche,
wobei zumindest ein Justagewert bereitgestellt wird,
wobei der die Ermittlung des zumindest einen Simulationswertes durch die Recheneinheit auch anhand des zumindest einen Justagewertes erfolgt.

11. Verfahren nach einem der vorangehenden Ansprüche,
wobei der Recheneinheit eine Protokoll-Queue mit mehreren MR-Pulssequenzen bereitgestellt wird,
wobei für jede der mehreren MR-Pulssequenzen zumindest ein Simulationswert durch die Recheneinheit ermittelt werden.

12. Verfahren nach Anspruch 11,
wobei anhand des Simulationswertes die MR-Pulssequenz, insbesondere mittels eines neuronalen Netzes, optimiert wird.

13. Recheneinheit, die ausgebildet ist, anhand einer MR-Pulssequenz und zumindest eines Patientenwertes zumindest einen Simulationswert zu ermitteln,
wobei die MR-Pulssequenz eine zeitliche Abfolge von mehreren HF-Pulsen und mehreren Gradientenpulsen umfasst,
wobei der zumindest eine Patientenwert eine Eigenschaft des Patienten beschreibt,
wobei der zumindest eine Simulationswert eine sicherheitsrelevante Größe bei einer Durchführung einer MR-Messung anhand der MR-Pulssequenz beschreibt,
wobei bei die Recheneinheit ferner ausgebildet ist, bei einer Ermittlung des zumindest einen Simulationswertes spezifische Eigenschaften der HF-Pulse und der Gradientenpulse der MR-Pulssequenz sowie deren zeitliche Abfolge zu berücksichtigen.

14. MR-Scanner mit einer Recheneinheit nach Anspruch 13.

15. Computerprogrammprodukt, welches ein Programm umfasst und direkt in einen Speicher einer Recheneinheit zur Ermittlung zumindest eines Simulationswertes ladbar ist, mit Programmmitteln, um ein Verfahren nach einem der Ansprüche 1 bis 12 auszuführen, wenn das Programm in der Recheneinheit ausgeführt wird.
